# EUROPEAN PATENT SPECIFICATION

(11) **EP 2 030 589 B1**
(45) Date of publication and mention of the grant of the patent: **06.04.2011**
(21) Application number: 08163256.4
(22) Date of filing: 29.08.2008
(51) Int. Cl.: A61C 9/00, A61K 6/10

(54) **A composite product for taking the impression of an edentulous arch**
Verbundprodukt zum Nehmen eines Abdrucks eines zahnlosen Zahnbogens
Produit composite pour prendre l'impression d'un arc édenté

(30) Priority: 03.09.2007 IT TO20070621
(43) Date of publication of application: 04.03.2009
(73) Proprietor: Major Prodotti Dentari S.P.A., 10024 Moncalieri (TO) (IT)
(72) Inventor: Preti, Giulio, 10024 Moncalieri (Torino) (IT); Gassino, Gianfranco, 10152 Torino (IT); Ceruti, Paola, 10137 Torino (IT); Rizzatti, Alessio, 14100 Asti (IT); Politi, Giorgio, 10135 Torino (IT); Gentile, Francesco, 10046 Poirino (Torino) (IT)
(74) Representative: Fioravanti, Corrado

(56) References cited:
- DE-C1- 4 304 421
- US-A- 5 011 407
- US-B1- 6 247 926

## Description

The present invention relates to a layered composite product which can be applied to an impression tray to take the impression of an edentulous arch.

At present, a dentist wishing to construct a prosthesis for an edentulous arch has to select the two most suitable impression trays from a set of impression trays of different sizes, one for taking the impression of the mandibular arch and one for the impression of the maxillary arch. A layer of alginate compound is applied to each of the selected impression trays; a preliminary impression is taken in this way, and is subsequently cast in plaster. This produces what is known as the "first model", on the basis of which the dental technician constructs two individual impression trays which are much more precise and closer to the morphology of the patient's maxillary and mandibular arches. The final impression is taken in these second impression trays in a second visit. This results in the two master models, on which the dental technician constructs what are known as the two "bases" from resin, on which are applied strips of wax (known as "valleys"), whose position and size simulate the dental arches.

Document US 6 847 926 B1 discloses an impression tray with a deformable material to form a first mold and a light curable material coupled to said with a bonding layer.

A primary object of the present invention is to reduce the clinical time required for the construction of the prostheses, and particularly to decrease the number of dental appointments and the discomfort for the patient, as well as the costs incurred by the patient. Another object of the invention is to simplify and optimize the laboratory operations for taking impressions of edentulous arches. A further object of the invention is to transfer the morphological data in a precise manner from the patient's oral cavity to the prosthesis support.

These and other objects and advantages, which will be made clearer below, are achieved according to the present invention by means of a composite product having the features stated in the appended claims.

Two preferred and non-limiting embodiments of the invention will now be described; reference will be made to the attached drawings, in which:
- Figure 1 is a schematic perspective view of a first embodiment of a layered composite product according to the invention; for convenience of representation, the proportions are not shown accurately;
- Figure 2 is a schematic view in vertical section of a step of the taking of the impression of an edentulous maxillary arch;
- Figure 3 is a schematic perspective view of a second embodiment of a layered composite product according to the invention.

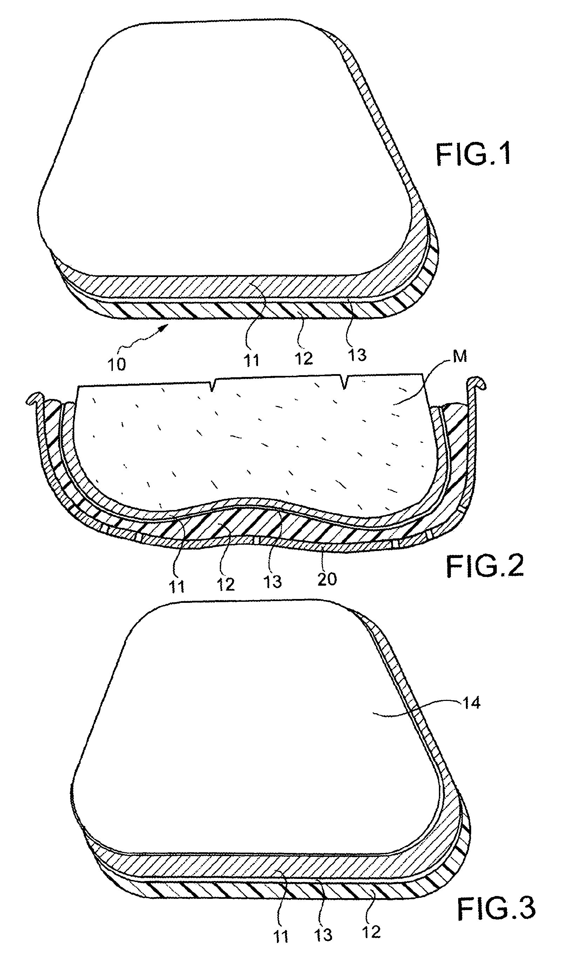

With reference initially to Figure 1, the number 10 indicates the whole of a layered composite product according to the invention which can be applied to a generic impression tray (indicated schematically by 20 in Figure 2) to take the impression of an edentulous arch. In the example illustrated and described herein, reference is made to taking the impression of a maxillary arch, and therefore terms and expressions such as "higher" and "lower" are not to be interpreted in a limiting sense. Clearly, the invention is equally applicable to taking an impression of an edentulous mandibular arch, and of more or less extensive portions of at least a part of an edentulous arch which may be either maxillary or mandibular.

The composite product 10 is composed of three layers superimposed on each other, namely a layer 11 of photopolymerizable material, a layer of thermoplastic material 12, and a separating layer 13, interposed between the photopolymerizable layer 11 and the thermoplastic layer 12.

The material forming the layer 11 is of a known type, being a composite resin which can be photopolymerized by exposure to ultraviolet light (wavelength 350-400 nm) or halogen light (wavelength 420-480 nm). For example, a photopolymerizable resin sheet for dental use such as the "Light Cure Tray", marketed by Major Prodotti Dentari S.p.A., of Moncalieri (TO), Italy, can be used to form this layer. Alternatively, the photopolymerizable material marketed by Zhermac S.p.A., of Badia Polesine (RO), Italy, under the brand name "Elite LC Tray" can be used. In all cases, the material is malleable and is able to continuously contact the mucosa of the maxillary (or mandibular) arch, so that it copies its shape and thus takes an impression of it. For guidance, the thickness of the photopolymerizable layer 11 may be in the range from 1 to 4 mm, and more preferably from 1.5 to 2 mm.

The layer of thermoplastic material 12, which primarily acts as a filler, is preferably made from wax or substances having a similar consistency to wax, which become malleable and plastic when heated to about 45-50°C, and which re-harden when cooled. For example, the wax for dental use called "Ceradent - cera azzurrina morbidissima", marketed by Industria Zingardi S.r.l., of Novi Ligure (AL), Italy, is particularly suitable. The thickness of the wax layer 12 may vary from 1 to 12 mm. It will be evident to those skilled in the art that different thicknesses can be used; since the function of this layer is to fill the space between the generic impression tray 20 and the maxillary arch M (Figure 2), the composite product 10 can be made with thermoplastic layers of different thicknesses, which the dentist can select from time to time according to the degree of reabsorption of the arch (maxillary or mandibular). Where the dimensions of the maxillary (or mandibular) arch are smaller, the thermoplastic layer 12 must be thicker.

The separating layer 13 is composed of an extendible elastic film of polymer material having a thickness of a few microns. The transparent film called "Cuki", marketed by Comital S.p.A., of Brandizzo (TO), Italy, has been found to be particularly suitable.

The composite product 10 is first heated, in water for example, to about 45-50°C. When the thermoplastic layer 12 has become sufficiently malleable and plastic, the dentist applies the composite product 10 to the impression tray 20, placing the thermoplastic layer 12 against the upper or "active" surface of the impression tray. The edges of the product 10 projecting beyond the impression tray 20 are cut off, and the impression of the edentulous arch is taken. These operations are carried out in a known way, and therefore they are not described here.

An important advantage of the thermoplastic layer 12 is that the dentist is free to take the impression again, if required in any particular case, in order to force the product 10 to copy the shape of the edentulous arch exactly. The dentist can extract the group composed of the impression tray 20 and the product 10 from the patient's oral cavity and reinsert it several times, after reheating the assembly if necessary by immersing it in hot water.

When the impression has been taken, the thermoplastic layer 12 is cooled, for example by immersing the impression tray with all of the layered product 10 in cold water.

When subjected to ultraviolet or halogen light, the layer 11 is polymerized, thus stably fixing the impression of the edentulous arch. When hardened, the polymerized layer is easy to detach cleanly from the thermoplastic layer 12, because of the separating film 13.

As will be appreciated, the final impression tray, formed by the photopolymerized layer, is produced in the course of a single appointment for the patient at the dentist's, instead of the two or three appointments required conventionally. It is easy to remove the wax of the layer 12 from the generic impression tray 20. The choice of the cellophane film as the impermeable separating layer between the photopolymerizable layer 11 and the thermoplastic layer 12 is particularly advantageous, since it facilitates the detachment of the photopolymerized layer without causing appreciable deformation of this layer.

The thermoplastic layer 12 not only adapts itself to fill the space between the generic impression tray and the edentulous arch, but also contributes to the retention of the shape impressed into the photopolymerizable layer until the latter layer is polymerized; it is easily removable from the various fastening apertures provided in conventional impression trays, and, as stated above, enables the dentist to take the impression in more than one operation by removing the product from the patient's mouth and modifying it manually until the ideal shape is obtained for taking the impression.

The embodiment illustrated schematically in Figure 3 differs from that of Figures 1 and 2 by the presence of a further impermeable surface film layer 14, which covers the outer surface of the photopolymerizable layer 11 in order to prevent the direct contact of the photopolymerizable material with the oral mucosa.

Clearly, the invention should not be considered to be limited to the embodiments described and illustrated herein, which represent only two examples of embodiment of the composite product. The invention can be applied in a similar way for taking impressions of portions or sectors of an edentulous arch, as will be evident to those skilled in the art. Finally, the particular types of material used to form each of the layers of the composite product according to the invention, and their thicknesses and the ratios of their dimensions, can be chosen from time to time according to requirements, without departure from the scope of the invention as defined by the following claims.

## Claims

1. A layered composite product (10) which can be applied to an impression tray (20) to take the impression of at least a part of an edentulous arch, comprising:
- a layer of photopolymerizable material (11),
- a layer of thermoplastic material (12),
- a separating layer (13) interposed between the layer of photopolymerizable material (11) and the layer of thermoplastic layer (12).

2. A composite product according to Claim 1, in which the layer of photopolymerizable material (11) comprises a composite resin which can be photopolymerized by exposure to ultraviolet light (wavelength 350-400 nm) or halogen light (wavelength 420-480 nm).

3. A composite product according to Claim 1, in which the thickness of the photopolymerizable layer (11) is in the range from about 1 to about 4 mm.

4. A composite product according to Claim 3, in which the thickness of the photopolymerizable layer (11) is in the range from about 1.5 to about 2.5 mm.

5. A composite product according to Claim 1, in which the layer of thermoplastic material (12) comprises wax of a substance having a consistency similar to that of wax.

6. A composite product according to Claim 5, in which the layer of thermoplastic material (12) becomes malleable and plastic when heated to about 45-50°C.

7. A composite product according to Claim 1, in which the thickness of the thermoplastic layer (12) is in the range from about 1 mm to about 12 mm.

8. A composite product according to Claim 1, in which the separating layer (13) is composed of an extendible elastic film of polymer material.

9. A composite product according to Claim 1, which also includes a surface layer (14) which covers the outer surface of the photopolymerizable layer (11).

10. A composite product according to Claim 9, in which the surface layer (14) is composed of an impermeable film.

## Patentansprüche

1. Geschichtetes Verbundprodukt (10), das auf einem Abdrucklöffel (20) aufgetragen werden kann, um den Abdruck zumindest eines Teil eines Gebisses aufzunehmen, umfassend:
- eine Schicht aus photopolymerisierbarem Material (11),
- eine Schicht aus thermoplastischem Material (12),
- eine Trennschicht (13), die zwischen der Schicht aus photopolymerisierbarem Material (11) und der Schicht aus thermoplastischem Material (12) angeordnet ist.

2. Verbundprodukt nach Anspruch 1, worin die Schicht aus photopolymerisierbarem Material (11) ein Verbundharz umfasst, das photopolymerisiert werden kann, indem es Ultraviolettlicht (Wellenlänge 350-400 nm) oder Halogenlicht (Wellenlänge 420-480 nm) ausgesetzt wird.

3. Verbundprodukt nach Anspruch 1, worin die Dicke der photopolymerisierbaren Schicht (11) im Bereich von etwa 1 bis etwa 4 mm liegt.

4. Verbundprodukt nach Anspruch 3, worin die Dicke der photopolymerisierbaren Schicht (11) im Bereich von etwa 1,5 bis etwa 2,5 mm liegt.

5. Verbundprodukt nach Anspruch 1, worin die Schicht aus thermoplastischem Material (12) ein Wachs aus einer Substanz umfasst, die eine ähnliche Konsistenz wie Wachs hat.

6. Verbundprodukt nach Anspruch 5, worin die Schicht aus thermoplastischem Material (12) form- und dehnbar wird, wenn sie auf etwa 45-50°C erhitzt wird.

7. Verbundprodukt nach Anspruch 1, worin die Dicke der thermoplastischen Schicht (12) im Bereich von etwa 1 mm bis etwa 12 mm liegt.

8. Verbundprodukt nach Anspruch 1, worin die Trennschicht (13) aus einem dehnbaren, flexiblen Polymermaterialfilm besteht.

9. Verbundprodukt nach Anspruch 1, das auch eine Oberflächenschicht (14) umfasst, welche die äußere Oberfläche der photopolymerisierbaren Schicht (11) bedeckt.

10. Verbundprodukt nach Anspruch 9, worin die Oberflächenschicht (14) aus einem undurchlässigen Film besteht.

## Revendications

1. Produit composite stratifié (10) qui peut être appliqué sur un porte-empreinte (20) pour prendre l'impression d'au moins une partie d'un arc édenté, comprenant :
- une couche de matériau photopolymérisable (11),
- une couche de matériau thermoplastique (12),
- une couche de séparation (13) interposée entre la couche de matériau photopolymérisable (11) et la couche de couche thermoplastique (12).

2. Produit composite selon la revendication 1, dans lequel la couche de matériau photopolymérisable (11) comprend une résine composite qui peut être photopolymérisée par exposition à une lumière ultraviolette (longueur d'onde 350-400 nm) ou à une lumière halogène (longueur d'onde 420-480 nm).

3. Produit composite selon la revendication 1, dans lequel l'épaisseur de la couche photopolymérisable (11) est dans l'intervalle d'environ 1 à environ 4 mm.

4. Produit composite selon la revendication 3, dans lequel l'épaisseur de la couche photopolymérisable (11) est dans l'intervalle d'environ 1,5 à environ 2,5 mm.

5. Produit composite selon la revendication 1, dans lequel la couche de matériau thermoplastique (12) comprend une cire d'une substance ayant une consistance similaire à celle de la cire.

6. Produit composite selon la revendication 5, dans lequel la couche de matériau thermoplastique (12) devient malléable et plastique lorsqu'elle est chauffée à environ 45-50 °C.

7. Produit composite selon la revendication 1, dans lequel l'épaisseur de la couche thermoplastique (12) est dans l'intervalle d'environ 1 mm à environ 12 mm.

8. Produit composite selon la revendication 1, dans lequel la couche de séparation (13) est composée d'un film élastique extensible de matériau à base de polymères.

9. Produit composite selon la revendication 1, qui comprend également une couche superficielle (14) qui recouvre la surface externe de la couche photopolymérisable (11).

10. Produit composite selon la revendication 9, dans lequel la couche superficielle (14) est composée d'un film imperméable.
